# EUROPEAN PATENT SPECIFICATION

(11) **EP 2 409 668 B1**
(45) Date of publication and mention of the grant of the patent: **07.11.2012**
(21) Application number: 11425198.6
(22) Date of filing: 20.07.2011
(51) Int. Cl.: A61F 2/00

(54) **A shape-memory type artificial sphincter**
Künstlicher Sphinkter mit Formgedächtnis
Sphincter artificiel à mémoire de forme

(30) Priority: 20.07.2010 IT RM20100400
(43) Date of publication of application: 25.01.2012
(73) Proprietor: Umbra Cuscinetti S.p.A., 06034 Foligno (PG) (IT)
(72) Inventor: Perni, Federico, Trevi (PG) (IT); Pizzoni, Luciano, Foligno (PG) (IT); Vairo, Giuseppe, Roma (IT); Enrico, Anna Chiara, Spoleto (PG) (IT); Porena, Massimo, Perugia (IT)
(74) Representative: Lanzoni, Luciano

(56) References cited:
- EP-A1- 2 092 912
- US-A- 6 162 238
- US-A1- 2004 243 236
- US-A1- 2009 012 350
- CHONAN S ET AL: "Development of an artificial urethral valve using SMA actuators", SMART MATERIALS AND STRUCTURES, IOP PUBLISHING LTD., BRISTOL, GB, vol. 6, no. 4, 1 August 1997 (1997-08-01), pages 410-414, XP020071873, ISSN: 0964-1726, DOI: DOI:10.1088/0964-1726/6/4/004

## Description

This invention relates to an artificial sphincter, preferably urethral.

An artificial sphincter represents in particular a device that can guarantee the closing and opening of the canal around which it is positioned. The insertion of a sphincter prosthesis allows control of the flow of fluid inside the specific canal, restoring the continence mechanisms present in a healthy subject.

This invention can therefore be applied in the field of biomedical engineering, and more specifically in the sector of active biomedical devices.

Urinary incontinence is a particularly widespread disorder, especially in the elderly, significantly impairing the quality of life of those who suffer from it.

Prior art includes various types of artificial sphincters, which can be surgically installed, that are able to overcome this problem and improve the quality of life of patients afflicted by this disorder.

The most common artificial sphincter, able to satisfy patient requirements in the long-term, consists of a prosthetic device based on a mechanical system coupled to a hydraulic pump apparatus.

More specifically, this devices comprises a tank, an active portion, generally installed in the urethral bulb or the neck of the bladder, with the aforesaid pump between them interconnected by means of an appropriate hydraulic circuit. Following a command given by the patient, the hydraulic pump apparatus generates the flow of a solution through the components of the device in order to vary the internal pressure and allow or prevent the outflow of urine.

Disadvantageously, this device presents numerous limitations due mainly to the hydraulic nature of the activation. Leakage of fluid from one of its components is a frequent occurrence, causing a drop in pressure and non-functioning of the device, requiring surgical intervention to replace/repair it.

In addition, the hydraulic cabling, the large number of components and their positioning in various zones of the patient's abdomen require particular assembly skill on the part of the surgeon and make the surgical procedure particularly invasive.

One important problem concerns the good manual dexterity which the patient must have to control the mechanism. Due to the advanced age of the patients in whom these devices are implanted, and in view also of the possible concomitance of psycho-motor disabilities, the aforesaid requirement of manual dexterity represents an extremely limiting restraint for their application.

The prior art also includes electromechanical devices that do not envisage the use of any hydraulic system.

Particular reference will be made to these devices since they constitute the closest prior art to the invention described herein.

In particular, the US patents 2006/0047180 and US 4556050 propose an electromechanical coupling through the use of active polymers or shape-memory materials.

Patent US 2006/0047180 describes a device presenting a support, which can be installed in the urethral canal, and an electro-active polymeric element that, following an appropriate command, can move a portion of the support in order to occlude or open this canal.

Disadvantageously, the electro-active polymer materials used to date are not specific enough and are consequently unreliable when used.

Patent US 4556050, on the other hand, is shaped like a "clamp", that is to say U-shaped, consisting of a plurality of overlapping plates and made from materials with different and complementary properties.

In particular, working outwards, the device comprises a plate made from soft material for contact with the patient's internal tissues, a shape-memory plate, a heating plate and an elastic plate which, in the released condition, holds the clamp on the urethral canal to occlude the passageway.

These plates overlap each other and are continually interconnected to prevent any relative movements.

Heating the shape-memory material changes its shape, thus opening the clamp and allowing outflow through the urethral canal.

Disadvantageously, in this multi-layer device the shape-memory plate is difficult to control due to its complex geometry and to the extensive hysteresis phenomena that arise following cyclical use. Other similar devices using shape-memory materials are described in published patents EP2092912, US 6162238, US2009/012350 and US 2004/243236.

In addition, the prior art devices take a fairly long time to recover their initial shape after urination.

In this context, the technical purpose which forms the basis of this invention is to propose an artificial sphincter that overcomes the above mentioned drawbacks of the prior art.

In particular, the aim of this invention is to provide an artificial sphincter with low hysteresis and extreme promptness.

Another aim of the invention is to provide an artificial sphincter which is easy to produce and safe to install.

The technical purpose indicated and the aims specified are substantially achieved by an artificial sphincter comprising the technical features described in one or more of the appended claims.

Further features and advantages of the invention are more apparent in the non-limiting description which follows of a preferred non-limiting embodiment of an artificial sphincter illustrated in the accompanying drawings, in which:

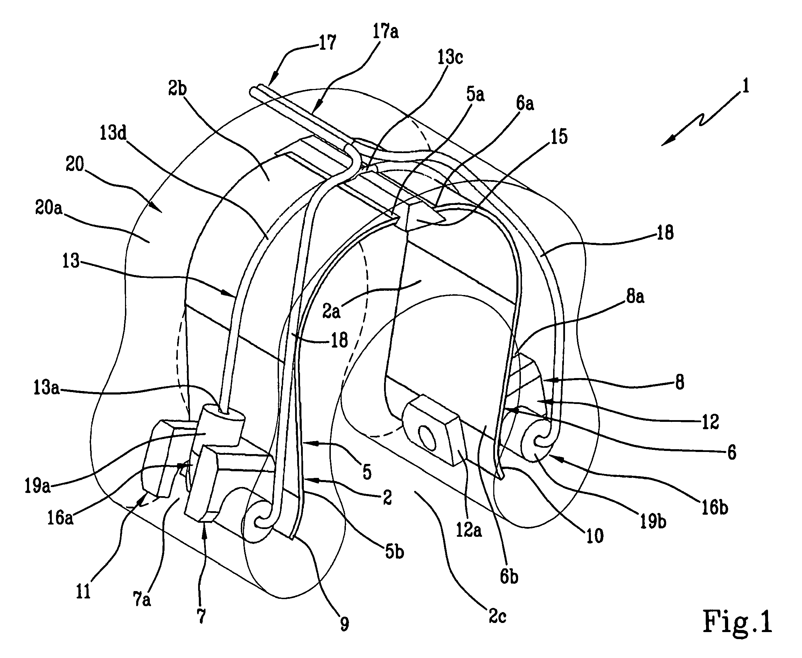

- figure 1 is a perspective view of an artificial sphincter according to this invention;

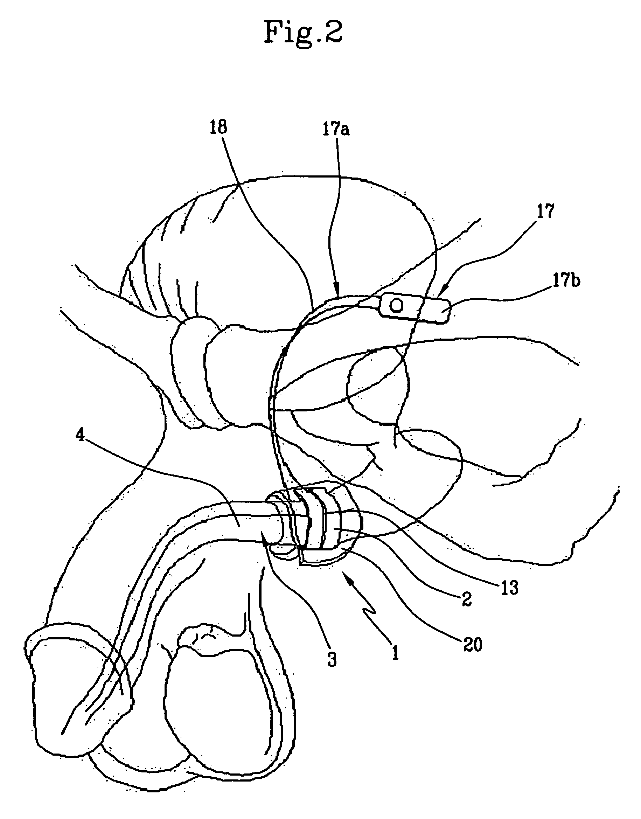

- figure 2 is a schematic view of the sphincter of figure 1 installed;

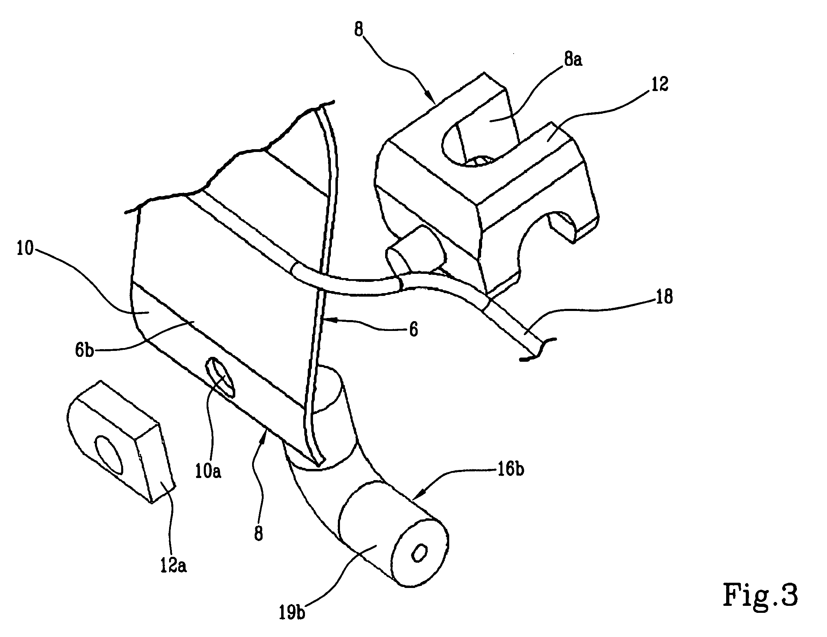

- figure 3 is an exploded view of a detail of the artificial sphincter of figure 1;
- figure 4 is a front view of a detail of another embodiment of an artificial sphincter according to this invention.

With reference to the accompanying drawings, the numeral 1 denotes an artificial sphincter according to this invention.

It should be noted that this description refers in particular to the application of the artificial sphincter 1 to the urethral canal; nevertheless other similar applications are fully compatible with this invention.

The artificial sphincter 1 comprises a gripping body 2, preferably made from biocompatible material, at least partially elastic so that it can be positioned around an anatomical duct 3, preferably of the urethral canal 4.

The gripping body 2 is preferably defined by a body folded back on itself to form a seat 2c to accommodate a portion of the aforesaid anatomical duct 3.

In the illustrated embodiment, the gripping body 2 presents a partially U-shaped conformation.

To fix the gripping body 2 it is thus sufficient to place it astride the duct 3.

In particular, the gripping body 2 comprises two walls 5, 6 which face each other and are elastically movable respectively approaching and/or distancing each other between a mutually proximal position, in which they occlude the anatomical duct 3, and a mutually distal position, in which they allow an outflow through the anatomical duct 3.

The seat 2c is defined by the walls 5, 6.

The walls 5, 6 are preferably elastically connected to each other at their respective connecting ends 5a, 6a.

In particular, the walls 5, 6 develop starting from their respective connecting ends 5a, 6a towards their free ends 5b, 6b.

These free ends 5b, 6b are therefore elastically movable respectively approaching and/or distancing each other, each along its own, preferably arched, trajectory.

In the preferred embodiment, the walls 5, 6 develop along a curved trajectory between the connecting end 5a, 6a and the free end 5b, 6b and are connected together so that their respective concave portions, defined by the curved shape, face each other.

In fact, as previously mentioned, the gripping body 2 has an arched shape presenting an intrados 2a, defined at least in part by faces of the walls 5, 6 opposite each other, and an extrados 2b opposite the intrados 2a.

Advantageously, this configuration makes the artificial sphincter 1 easy to assemble and install.

In fact, the intrados 2a defines the seat 2c which accommodates the anatomical duct 3, preferably the urethral canal 4.

The gripping body 2 therefore substantially presents a clamp configuration which facilitates its application around the anatomical duct 3.

The walls 5, 6 are preferably specular in order to give the gripping body 2 a substantially symmetrical structure.

Each wall 5, 6 preferably comprises at least one elastic portion.

Even more preferably, the walls 5, 6 are totally made from elastic material, preferably a biocompatible metal material.

In view of the above, the gripping body 2 substantially defines an arch-shaped spring, presenting two flexible sides which can move towards and away from each other, that is to say the walls 5, 6.

When the spring is in the released condition, the walls 5, 6 of the gripping body 2 are preferably in the proximal position.

In other words, the gripping body 2 is normally closed, that is to say the walls 5, 6 press against the sides of the duct 3, exercising appropriate compression in a direction preferably at right angles to the axis of the duct. This results in an elliptical geometry of the urethral canal, so that its inner walls can meet each other in a stationary condition.

The proposed geometry of the gripping body makes it possible to foresee different preloading levels (by simply varying the degree of opening at rest), without altering the pressure action and without changing the regularity of the artificial and tissue surfaces in contact with each other.

The front dimensions of the device can be defined on the basis of the specific anatomical characteristics of the patient, easily obtaining the definition of different size devices.

The gripping body 2 also presents two projections 7, 8 that develop away from each other, each starting from a respective wall 5, 6.

In other words, each wall 5, 6 is associated with a respective projection 7, 8 protruding from it away from the seat 2c.

These projections 7, 8 develop transversely to the walls 5, 6 in proximity to the free ends 5b, 6b, giving the gripping body 2 a "Ω" shape.

It should be noted that the term transversal means that the projections 7, 8 develop starting from the respective free end 5b, 6b along a different direction to the direction of development of the wall close to its free end 5b, 6b.

In a first embodiment (not shown in the figures), the projections 7, 8 are totally defined by fins made as one with the walls 5, 6 and are their natural continuation.

In another embodiment (not shown), the projections 7, 8 are defined by connecting bodies mounted directly on the walls.

In the illustrated embodiment, each projection 7, 8 comprises a respective fin 9, 10 made as one with the walls 5, 6 and divergent to them.

In addition, each fin 9, 10 is coupled with a connecting body 11, 12 projecting laterally to it.

The connecting body 11, 12 is preferably a separate element from the respective wall 5, 6 and is mounted on it by means of mechanical fastenings 11a, 12a.

In particular, the fins 9, 10 each present a respective hole 10a through which the connecting body 11, 12 is hinged and fixed by means of the said mechanical fastening 12a.

Each projection 7, 8 preferably presents a through slot 7a, 8a, whose function will be clarified further on in this description.

In the illustrated embodiment, the slot 7a, 8a is made in the connecting body 11, 12. In other embodiments, not illustrated, the slot can be cut in the fin.

The artificial sphincter 1 also comprises a switching element 13 connected to the gripping body 2 and selectively deformable between a first configuration, in which it maintains the walls 5, 6 in the proximal position, and a second configuration, in which it maintains the walls 5, 6 in the distal position.

Since the gripping body 2 is normally closed, the first configuration of the switching element 13 represents a passive condition, while the second configuration represents an active condition.

The switching element 13 can thus be activated to switch from the first to the second configuration in order to allow the outflow through the duct 3, and de-activated to switch from the second to the first configuration to occlude the passageway by the action of the gripping body.

The switching element 13 is preferably at least partially deformable in order to switch from the first to the second configuration.

In addition, the switching element 13 is at least partially, and preferably completely, of the shape-memory type.

More specifically, the switching element 13 is preferably made from Shape Memory Alloy, otherwise known as SMA.

The main characteristic of such alloys is that they are able to regain a predetermined macroscopic shape by merely changing the temperature or the level of stress applied. In other words, these materials are able to undergo reversible crystallographic transformations, according to the tension and heat status.

By way of example, the switching element 13 is at least partially, and preferably completely, made from a nickel-titanium alloy.

However, other shape memory alloys can also be used, making it possible to achieve the same results.

The change in temperature causes the switch from the first to the second configuration of the switching element 13.

The switching element 13 presents a substantially wire-like shape and is connected to the gripping body 2 at the projections 7, 8, preferably transversely to them.

In other words, the switching element 13 is a wire connected to the gripping body 2 at the projections 7, 8, and activated on these projections 7, 8 along a direction transversely to them.

In particular, the switching element 13 presents two junction portions 13a, 13b with the gripping body 2, positioned at the projections 7, 8.

These junction portions 13a, 13b are preferably at opposite ends of the switching element 13.

Due to the wire-like shape of the switching element 13, a variation in temperature involves a variation in the only significant dimension of this element, that is to say its length.

In other words, the length of the wire-like switching element 13 varies according to its temperature.

In the preferred embodiment, the length of the wire-like switching element 13 in the first configuration is greater than its length in the second configuration.

The rise in temperature of the switching element 13 is preferably achieved by Joule effect, as will be clarified in more detail below.

The switching element 13 is therefore preferably insulated thermally and electrically from the outside.

Advantageously, since the switching element 13 is transversely connected to the projections 7, 8, a change in the length of the wire causes a transverse pulling action on the projections 7, 8 which tends to move them away from each other.

In addition, the wire-like shape of the switching element 13 causes the heat to dissipate rapidly with a consequent return from the second to the first configuration, with notable advantages for the patient.

The switching element 13 preferably runs at least in part along the walls 5, 6 of the gripping body 2.

More specifically, the switching element 13 is positioned on the extrados 2b of the gripping body 2.

In other words, the switching element 13 develops around the gripping body between the projections 7, 8.

The pulling action therefore takes place from the free end 5b, 6b towards the connecting end 5a, 6a of each wall 5, 6.

In addition, the switching element 13 presents at least one free portion 13d, situated between the two junction portions 13a, 13b and freely resting on the walls 5, 6.

In other words, at least one portion of the switching element 13 rests freely on the walls 5, 6, that is to say it is not directly constrained to the wall.

More specifically, during the lengthening and/or shortening of he switching element 13, the free portion 13d slides along the walls 5, 6 of the gripping body 2.

The change in length of the switching element 13 is thus concentrated in the junction portions 13a, 13b and the pulling action is concentrated on the projections 7, 8. Consequently, since the projections 7, 8 are transversal to the walls 5, 6, the pulling action corresponds to a reaction bending moment which tends to move the walls 5, 6 away from each other.

In particular, the intensity of the moment in proximity to the free end 5b, 6b of each wall 5, 6 depends on the distance between the point of application of the pulling action, that is to say the connecting point between the switching element 13 and the projections 7, 8 and the connecting point between the projections 7, 8 and the walls 5,6.

The greater this bending moment, the wider the "opening" of the walls 5, 6.

The switching element 13 preferably presents an additional junction portion 13c connected to the extrados 2b of the gripping body 2.

Advantageously, the three-point connection makes it possible to avoid the dislocation of the switching element 13, maintaining a high level of deformation freedom.

The additional junction portion 13c is preferably connected to the gripping body 2 at a point substantially equidistant from the junction portions 13a, 13b. Advantageously, the shortening and/or lengthening of the switching element thus initiates an identical action on both projections 7, 8.

Due to the substantially symmetrical shape of the gripping body 2, the additional junction portion 13c is preferably positioned on a summit 15 of the extrados 2b.

In an alternative embodiment (not shown), the extrados 2b is equipped with a system of through holes in which the switching element 13 can slide during switching between the first and the second configuration.

The connection between the switching element 13 and the projections 7, 8 preferably takes place through a mechanical fastening.

In the illustrated embodiment, each junction portion 13a, 13b of the switching element 13 is inserted in the slot 7a, 8a of the respective projection 7, 8.

For this purpose, each junction portion 13a, 13b comprises a stopping body 16a, 16b to prevent these junction portions 13a, 13b slipping out through the slot 7a, 8a during the switch from the first to the second configuration.

In fact, each junction portion 13a, 13b inserted in the respective slot 7a, 8a during the switch from the first to the second configuration of the switching element 13 would tend to slip out of the slot 7a, 8a.

At this point, the stopping body 16a, 16b engages the projection 7, 8 preventing the switching element from slipping out and triggering the pulling action on the projection 7, 8.

In addition, the switching element 13 is preferably positioned along the center line of the extrados 2b.

Advantageously, this balances the action performed by the switching element 13. The artificial sphincter 1 also preferably comprises a plurality of switching elements 13 alongside each other and operating in parallel.

Advantageously, the use of several duplicate wire-like elements can ensure, even if one of them is damaged, the correct functioning of the artificial sphincter 1.

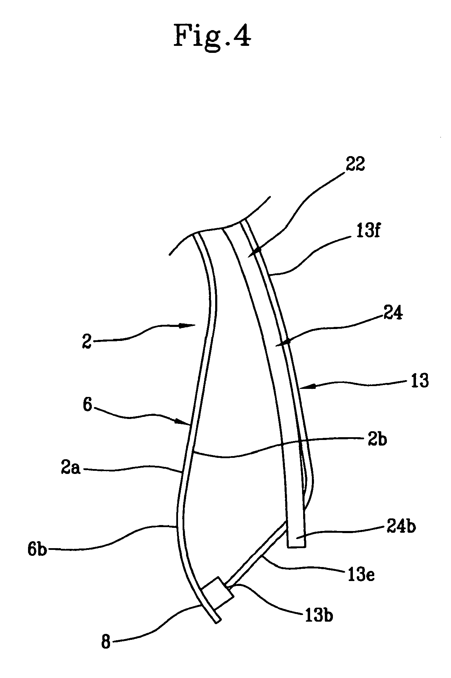

With reference to figure 4, the artificial sphincter 1 preferably comprises at least one spacer body 22 active on the switching element 13 and associated with each wall 5, 6 of the gripping body 2 to maintain said switching element 13 transverse to the respective projection 7, 8.

In particular, the spacer body 22 moves the switching element 13 away from each projection 7, 8, at least in proximity to each junction portion 13a, 13b to maintain said switching element 13 transverse to the respective projection 7, 8.

In other words, the spacer body 22 is active on the switching element 13 in proximity to the junction portions 13a, 13b to maintain the switching element 13 as close as possible at 90° to the projections 7, 8.

Advantageously, this makes it possible to maximize the action of the switching element 13 on the gripping body 2 since just a slight shortening leads to a significant "opening" of the gripping body 2.

In fact, this configuration increases the working force component to generate an "opening" moment of the gripping body 2.

The spacer body 22 preferably comprises at least one arm 24 connected to each wall 5, 6 of the gripping body 2.

More specifically, each arm 24 develops away from the wall 5, 6 between a first end (not shown) connected to the wall 5, 6 itself, and a second end 24b facing the projection 7, 8 preferably at a predetermined distance from it.

In addition, the switching element 13 is preferably maintained in traction between the second end 24b of each arm 24 and the respective projection 7, 8.

In other words, the switching element 13 presents two end portions 13e each developing in traction between the projection 7, 8 and the second end 24b of the arm 24.

It should be noted that each end portion 13e comprises the respective junction portion 13a, 13b.

The spacer body 22 is preferably made from a material with a greater bending rigidity with respect to the gripping body 2 so that during the shortening of the switching element 13 the walls 5, 6 of the gripping body 2 bend and not the arms 24 of the spacer body 22.

In proximity to each second end 24b of the arm, the spacer body 22 also presents an opening or slot (not shown) through which the switching element 13 passes.

As already mentioned, in order to obtain the heating of the switching element 13, the artificial sphincter 1 comprises a power supply system 17.

The power supply system 17 comprises a voltage generator 17b and a wiring system 17a connected to the switching element 13, at its opposite ends, to transfer the current to the switching element 13.

In fact, a flow of electrical current, preferably of low intensity, through the wire-like switching element 13 causes it to heat, by Joule effect, and thus, due to phase transformation, modifies its crystallographic organisation and switches it from the first to the second configuration.

The heating of the wire-like element is therefore direct and not induced by contact with a heating element.

In a first embodiment, the voltage generator 17b comprises a battery, which can be the rechargeable type and is appropriately insulated to allow it to be implanted inside the patient.

Alternatively, it is possible to generate percutaneous transmission of energy by inducing a current between two windings, one inside and the other outside the patient's body.

As far as the wiring 17a is concerned, this comprises a system of wire 18 and cables, insulated and mechanically protected by means of biocompatible and corrosion-resistant materials.

These wires 18 are preferably connected to the ends 13a, 13b of the switching element 13.

In particular, in the illustrated embodiment, a connector 19a, 19b connects each end 13a, 13b of the switching element 13 to the wire 18 of the wiring system 17a.

Each connector 19a, 19b preferably acts as a stopping body 16a, 16b and is engaged with the projection 7, 8.

The configuration of the stopping body can be as illustrated (L-shaped) or U, T or C-shaped in order to conform to the component (7, 8) on which it operates.

In the illustrated embodiment, each connector 19a, 19b engages with the respective connecting body 11, 12 and is positioned in a housing in the connecting body.

In addition, the artificial sphincter 1 comprises an activation system (not shown) to activate or de-activate the voltage generator 17b and move the switching element from the first to the second configuration and vice versa.

In a first embodiment the activation system comprises a wireless device associated with the source.

Alternatively, the artificial sphincter 1 envisages the use of a two-way switch connected by a circuit to the power supply system 17.

This activation system is also preferably installed in the patient so as to be easily accessible and controllable, especially in consideration of the often advanced age of those who need this type of apparatus.

In addition, the artificial sphincter 1 preferably comprises a containment case 20, preferably made of elastomeric material, of the gripping body 2 and of the switching element 13.

More specifically, the containment case 20 is made from flexible biocompatible material to carry out both mechanical and biological functions.

The preferably elastomeric material of the containment case 20 can, for example, be silicone, polyurethane, teflon® or any polymer that can respond to the above-defined requirements.

This containment case 20 is positioned around the gripping body 2 to provide, on one hand, appropriate thermo-electric insulation with the surrounding tissues and charge distribution, and on the other hand to minimize the interaction with the surrounding tissues.

In the illustrated embodiment, the containment case 20 is at least partly defined by a layer 20a enclosing the gripping body 2.

In other words, the layer 20a adapts to the profile of the intrados 2a and the extrados 2b of the gripping body 2.

In addition, the case 20 presents at least two projecting edges (not shown) to enable the suturing of the case 20 itself to the anatomical duct 3 or the surrounding tissues. In other embodiments (not shown), the case presents a substantially ring-like shape to allow complete enclosure of the anatomical duct 3.

In this event, the case is wrapped around the anatomical duct 3 during the surgical operation and secured by suturing or a loop system.

The invention achieves the above mentioned aims and has important advantages.

In fact, the presence of a wire-like switching element which acts transversely to the projections makes the switch from one configuration of the artificial sphincter to the other simple and fast.

In particular, a slight shortening of the switching element causes the walls to move far enough apart to allow the outflow through the anatomical duct.

In addition, the good connection at the projections concentrates the pulling action on the projections, increasing efficiency.

Moreover, the use of a wire-like element speeds up the heating and cooling of the switching element and consequently speeds up the switch between the first and second configurations.

Finally, the presence of a biocompatible case which can be secured to the patient's internal tissues by suturing makes the position of the artificial sphincter stable over time, increasing its lifetime efficiency.

## Claims

1. An artificial sphincter comprising:
- a gripping body (2) presenting two walls (5, 6) which face each other to define a seat (2c) for receiving an anatomical duct (3); said walls (5, 6) being elastically movable respectively approaching and/or distancing each other between a mutually proximal position, in which they occlude the anatomical duct (3), and a mutually distal position, in which they allow a flow through the anatomical duct (3);
- at least one switching element (13), of the shape-memory type, connected to the gripping body (2) and selectively deformable between a first configuration, in which it maintains the walls (5, 6) in proximal position, and a second configuration, in which it maintains the walls (5, 6) in distal position; wherein the switching element (13) presents a substantially wire-like shape **characterised in that** the gripping body (2) presents two projections (7, 8) that develop away from each other, each starting from a respective wall (5, 6); said switching element (13) being connected to the gripping body (2) at the projections (7, 8) transversely thereto.

2. An artificial sphincter as claimed in claim 1, **characterised in that** said switching element (13) can be activated to switch from the first to the second configuration and de-activated to switch from the second to the first configuration; the length of the wire-like switching element (13) being greater in the first configuration than in the second configuration.

3. An artificial sphincter as claimed in any of the previous claims, **characterised in that** the walls (5, 6) are elastically connected to each other at respective connecting ends (5a, 6a); said switching element (13) being positioned at least in part along said walls (5, 6) of the gripping body (2) to move them away from each other when it is in the first configuration.

4. An artificial sphincter as claimed in any of the previous claims, **characterised in that** said walls (5, 6) define an arched shape of the gripping body (2); said switching element (13) being positioned on an extrados (2b) of said gripping body (2).

5. An artificial sphincter as claimed in claim 3 or 4, **characterized in that** the projections (7, 8) are positioned in proximity to the free ends (5b, 6b) of the walls (5, 6).

6. An artificial sphincter as claimed in any of the previous claims, **characterised in that** the switching element (13) develops at least in part between two junction portions (13a, 13b) connected to the gripping body (2); said switching element (13) presenting at least one free portion situated between said two junction portions (13a, 13b) freely bearing on the walls (5, 6).

7. An artificial sphincter as claimed in claim 6, **characterised in that** each projection (7, 8) presents a respective slot (7a, 8a) into which the junction portions (13a, 13b) of the switching element (13) are inserted; said junction portions (13a, 13b) comprising a stopping body (16, 16b) able to engage the projection (7, 8) to prevent the withdrawal of said junction portions (13a, 13b) through the slot (7a, 8a).

8. An artificial sphincter as claimed in claim 7, **characterised in that** each stopping body (16a, 16b) comprises a connector (19a, 19b) connected to connect the switching element (13) to a voltage generator (17b).

9. An artificial sphincter as claimed in any of the previous claims, **characterised in that** it comprises at least one spacer body (22) active on the switching element (13) and associated to each wall (5, 6) of the gripping body (2); said spacer body (22) moving the switching element (13) away from each projection (7, 8) at least in proximity to each junction portion (13a, 13b) to maintain said switching element (13) transverse to the respective projection (7, 8).

10. An artificial sphincter as claimed in claim 9, **characterised in that** the spacer body (22) comprises at least one arm (24) connected to each wall (5, 6) of the gripping body (2), each developing away from said wall (5, 6), between a first end, connected to the wall (5, 6) itself, and a second end (24b) facing the projection (7, 8); said switching element (13) being maintained in traction between the second end (24b) of each arm (24) and the projection (7, 8).

11. An artificial sphincter as claimed in any of the previous claims, **characterised in that** each projection (7, 8) comprises a connecting body (11, 12) distinct from the respective wall (5, 6) and mounted on said wall (5, 6) by means of a respective mechanical fastening (12a).

12. An artificial sphincter as claimed in any of the previous claims, **characterised in that** it comprises a voltage generator (17b) connected to opposite ends of the switching element (13) and able to be activated to raise the temperature of said switching element (13) to bring it from the first to the second configuration.

13. An artificial sphincter as claimed in any of the previous claims, **characterised in that** it comprises one containment case (20), preferably made of elastomeric material, of the gripping body (2) and of the switching element (13) positioned around said gripping body (2) and said switching element (13) to provide a thermoelectric insulation with the surrounding tissues.

14. An artificial sphincter as claimed in claim 13, **characterised in that** the case (20) presents at least two projecting edges to enable the suture of the case (20) itself.

15. An artificial sphincter as claimed in any of the previous claims, **characterised in that** it comprises two or more of said switching elements (13) positioned and active in mutually parallel manner.

## Patentansprüche

1. Künstlicher Sphinkter, umfassend:
- einen Greifkörper (2), aufweisend zwei Wände (5, 6), die einander zugewandt sind, um einen Sitz (2c) zur Aufnahme einer anatomischen Leitung (3) zu definieren; wobei sich die Wände (5, 6) elastisch beweglich jeweils zwischen einer Position proximal zueinander, in der sie die anatomische Leitung (3) okkludieren, und einer Position distal zueinander, in der sie einen Durchfluss durch die anatomische Leitung (3) gestatten, einander annähern und/oder entfernen;
- mindestens ein Umschaltelement (13) mit Formgedächtnis, das mit dem Greifkörper (2) verbunden ist und zwischen einer ersten Konfiguration, in der es die Wände (5, 6) in proximaler Position hält und einer zweiten Konfiguration, in der es die Wände (5, 6) in distaler Position hält, selektiv verformbar ist; wobei das Umschaltelement (13) eine im Wesentlichen drahtähnliche Form aufweist,
**dadurch gekennzeichnet, dass** der Greifkörper (2) zwei Vorsprünge (7, 8) aufweist, die sich voneinander weg erstrecken, wobei jeder an einer entsprechenden Wand (5, 6) beginnt, wobei das Umschaltelement (13) mit dem Greifkörper (2) an den Vorsprüngen (7, 8) transversal zu diesen verbunden ist.

2. Künstlicher Sphinkter nach Anspruch 1, **dadurch gekennzeichnet, dass** das Umschaltelement (13) aktiviert werden kann, um von der ersten in die zweite Konfiguration umzuschalten und deaktiviert werden kann, um von der zweiten in die erste Konfiguration umzuschalten; wobei die Länge des drahtähnlichen Umschaltelements (13) in der ersten Konfiguration größer als in der zweiten Konfiguration ist.

3. Künstlicher Sphinkter nach einem der vorangehenden Ansprüche, **dadurch gekennzeichnet, dass** die Wände (5, 6) an entsprechenden Verbindungsenden (5a, 6a) elastisch miteinander verbunden sind; wobei das Umschaltelement (13) zumindest teilweise entlang der Wände (5, 6) des Greifkörpers (2) positioniert ist, um sie voneinander weg zu bewegen, wenn es in der ersten Konfiguration ist.

4. Künstlicher Sphinkter nach einem der vorangehenden Ansprüche, **dadurch gekennzeichnet, dass** die Wände (5, 6) eine Bogenform des Greifkörpers (2) definieren; wobei das Umschaltelement (13) auf einem Bogenrücken (2b) des Greifkörpers (2) positioniert ist.

5. Künstlicher Sphinkter nach Anspruch 3 oder 4, **dadurch gekennzeichnet, dass** die Vorsprünge (7, 8) in der Nähe der freien Enden (5b, 6b) der Wände (5, 6) positioniert sind.

6. Künstlicher Sphinkter nach einem der vorangehenden Ansprüche, **dadurch gekennzeichnet, dass** sich das Umschaltelement (13) zumindest teilweise zwischen zwei zusammenführenden Abschnitten (13a, 13b) erstreckt, die mit dem Greifkörper (2) verbunden sind; wobei das Umschaltelement (13) mindestens einen freien Abschnitt aufweist, der sich zwischen den zwei zusammenführenden Abschnitten (13a, 13b) frei an den Wänden (5, 6) anliegend befindet.

7. Künstlicher Sphinkter nach Anspruch 6, **dadurch gekennzeichnet, dass** jeder Vorsprung (7, 8) einen entsprechenden Schlitz (7a, 8a) aufweist, in den die zusammenführenden Abschnitte (13a, 13b) des Umschaltelements (13) eingefügt sind; wobei die zusammenführenden Abschnitte (13a, 13b) einen Anhaltekörper (16, 16b) umfassen, der zu einem Eingriff in den Vorsprung (7, 8) fähig ist, um ein Zurückziehen der zusammenführenden Abschnitte (13a, 13b) durch den Schlitz (7a, 8a) zu verhindern.

8. Künstlicher Sphinkter nach Anspruch 7, **dadurch gekennzeichnet, dass** jeder Anhaltekörper (16a, 16b) ein Anschlussstück (19a, 19b) umfasst, das zur Verbindung des Umschaltelements (13) mit einem Spannungsgenerator (17b) angeschlossen ist.

9. Künstlicher Sphinkter nach einem der vorangehenden Ansprüche, **dadurch gekennzeichnet, dass** er mindestens einen Distanzkörper (22) umfasst, der auf das Umschaltelement (13) einwirkt und an jede Wand (5, 6) des Greifkörpers (2) anschließt, wobei der Distanzkörper (22) das Umschaltelement (13) von jedem Vorsprung (7, 8) zumindest in der Nähe von jedem zusammenführenden Abschnitt (13a, 13b) wegbewegt, um das Umschaltelement (13) transversal zum entsprechenden Vorsprung (7, 8) zu halten.

10. Künstlicher Sphinkter nach Anspruch 9, **dadurch gekennzeichnet, dass** der Distanzkörper (22) mindestens einen Arm (24) umfasst, der mit jeder Wand (5, 6) des Greifkörpers (2) verbunden ist, der sich jeweils von der Wand (5, 6) zwischen einem ersten Ende, das mit der Wand (5, 6) verbunden ist, und einem zweiten Ende (24b), das dem Vorsprung (7, 8) zugewandt ist, weg erstreckt; wobei das Umschaltelement (13) durch Zugkraft zwischen dem zweiten Ende (24b) eines jeden Arms (24) und dem Vorsprung (7, 8) gehalten wird.

11. Künstlicher Sphinkter nach einem der vorangehenden Ansprüche, **dadurch gekennzeichnet, dass** jeder Vorsprung (7, 8) einen Verbindungskörper (11, 12) umfasst, der von der entsprechenden Wand (5, 6) abgesetzt und an der Wand (5, 6) mithilfe einer entsprechenden mechanischen Befestigung (12a) angebracht ist.

12. Künstlicher Sphinkter nach einem der vorangehenden Ansprüche, **dadurch gekennzeichnet, dass** er einen Spannungsgenerator (17b) umfasst, der mit entgegengesetzten Enden des Umschaltelements (13) verbunden ist und aktiviert werden kann, um die Temperatur des Umschaltelements (13) zu erhöhen, um es von der ersten in die zweite Konfiguration zu bringen.

13. Künstlicher Sphinkter nach einem der vorangehenden Ansprüche, **dadurch gekennzeichnet, dass** er eine vorzugsweise aus einem Elastomermaterial hergestellte Aufnahmeumhüllung (20) des Greifkörpers (2) und des Umschaltelements (13) aufweist, die um den Greifkörper (2) und das Umschaltelement (13) herum positioniert sind, um eine thermoelektrische Isolierung mit den umgebenden Geweben bereitzustellen.

14. Künstlicher Sphinkter nach Anspruch 13, **dadurch gekennzeichnet, dass** die Umhüllung (20) mindestens zwei vorspringende Kanten aufweist, um das Annähen der Umhüllung (20) zu ermöglichen.

15. Künstlicher Sphinkter nach einem der vorangehenden Ansprüche, **dadurch gekennzeichnet, dass** er ein oder mehrere Umschaltelemente (13) umfasst, die parallel zueinander positioniert und wirksam sind.

## Revendications

1. Sphincter artificiel comprenant :
- un organe de préhension (2) comportant deux parois (5, 6) faisant face l'une à l'autre pour définir un logement (2c) pour la réception d'un conduit anatomique (3) ; lesdites parois (5, 6) étant élastiquement mobiles, se rapprochant et/ou s'éloignant respectivement l'une de l'autre entre une position mutuellement proximale, dans laquelle elles occluent le conduit anatomique (3), et une position mutuellement distale, dans laquelle elles permettent à un écoulement de passer à travers le conduit anatomique (3) ;
- au moins un élément de commutation (13), à mémoire de forme, relié à l'organe de préhension (2) et étant déformable, de façon sélective, entre une première configuration, dans laquelle il maintient les parois (5, 6) dans une position proximale, et une seconde configuration, dans laquelle il maintient les parois (5, 6) dans une position distale ; dans lequel l'élément de commutation (13) a en substance la forme d'un fil **caractérisé en ce que** l'organe de préhension (2) comporte deux saillies (7, 8) qui se développent en s'éloignant l'une de l'autre, chacune commençant à partir d'une paroi respective (5, 6) ; ledit élément de commutation (13) étant relié à l'organe de préhension (2) au niveau des saillies (7, 8) transversalement à ces dernières.

2. Sphincter artificiel selon la revendication 1, **caractérisé en ce que** ledit élément de commutation (13) peut être activé pour passer de la première à la seconde configuration et désactivé pour passer de la seconde à la première configuration ; la longueur de l'élément de commutation filiforme (13) étant plus grande dans la première configuration que dans la seconde.

3. Sphincter artificiel selon l'une quelconque des revendications précédentes, **caractérisé en ce que** les parois (5, 6) sont élastiquement reliées l'une à l'autre à des extrémités de liaison respectives (5a, 6a) ; ledit élément de commutation (13) étant positionné au moins en partie le long desdites parois (5, 6) de l'organe de préhension (2) pour les éloigner l'une de l'autre lorsqu'il se trouve dans la première configuration.

4. Sphincter artificiel selon l'une quelconque des revendications précédentes, **caractérisé en ce que** lesdites parois (5, 6) définissent une forme cambrée de l'organe de préhension (2) ; ledit élément de commutation (13) étant positionné sur un extrados (2b) dudit organe de préhension (2).

5. Sphincter artificiel selon les revendications 3 ou 4, **caractérisé en ce que** les saillies (7, 8) sont positionnées à proximité des extrémités libres (5b, 6b) des parois (5, 6).

6. Sphincter artificiel selon l'une quelconque des revendications précédentes, **caractérisé en ce que** l'élément de commutation (13) se développe au moins en partie entre deux parties de jonction (13a, 13b) reliées à l'organe de préhension (2) ; ledit élément de commutation (13) comportant au moins une partie libre située entre lesdites parties de jonction (13a, 13b) librement en appui sur les parois (5, 6).

7. Sphincter artificiel selon la revendication 6, **caractérisé en ce que** chaque saillie (7, 8) comporte une fente respective (7a, 8a) dans laquelle sont insérées les parties de jonction (13a, 13b) de l'élément de commutation (13) ; lesdites parties de jonction (13a, 13b) comprenant un organe d'arrêt (16, 16b) apte à engager la saillie (7, 8) pour empêcher le retrait desdites parties de jonction (13a, 13b) à travers la fente (7a, 8a).

8. Sphincter artificiel selon la revendication 7, **caractérisé en ce que** chaque organe d'arrêt (16a, 16b) comprend un connecteur (19a, 19b) relié pour relier l'élément de commutation (13) à un générateur de tension (17b).

9. Sphincter artificiel selon l'une quelconque des revendications précédentes, **caractérisé en ce qu'**il comprend au moins un organe d'espacement (22) actif sur l'élément de commutation (13) et associé à chaque paroi (5, 6) de l'organe de préhension (2) ; ledit organe d'espacement (22) éloignant l'élément de commutation (13) de chaque saillie (7, 8) au moins à proximité de chaque partie de jonction (13a, 13b) afin de maintenir ledit élément de commutation (13) en position transversale par rapport à la saillie respective (7, 8).

10. Sphincter artificiel selon la revendication 9, **caractérisé en ce que** l'organe d'espacement (22) comprend au moins un bras (24) relié à chaque paroi (5, 6) de l'organe de préhension (2), chacun se développant en s'éloignant de ladite paroi (5, 6), entre une première extrémité, reliée à la paroi (5, 6) elle-même, et une seconde extrémité (24b) faisant face à la saillie (7, 8) ; ledit élément de commutation (13) étant maintenu en traction entre la seconde extrémité (24b) de chaque bras (24) et la saillie (7, 8).

11. Sphincter artificiel selon l'une quelconque des revendications précédentes, **caractérisé en ce que** chaque saillie (7, 8) comprend un organe de liaison (11, 12) distinct de la paroi respective (5, 6) et monté sur ladite paroi (5, 6) par le biais d'une fixation mécanique respective (12a).

12. Sphincter artificiel selon l'une quelconque des revendications précédentes, **caractérisé en ce qu'**il comprend un générateur de tension (17b) relié aux extrémités opposées de l'élément de commutation (13) et capable d'être activé pour élever la température dudit élément de commutation (13) pour le faire passer de la première à la seconde configuration.

13. Sphincter artificiel selon l'une quelconque des revendications précédentes, **caractérisé en ce qu'**il comprend une enveloppe de confinement (20), fabriquée de préférence dans un matériau élastomère, de l'organe de préhension (2) et de l'élément de commutation (13) positionnée autour dudit organe de préhension (2) et dudit élément de commutation (13) afin de garantir une isolation thermoélectrique avec les tissus environnant.

14. Sphincter artificiel selon la revendication 13, caractérisé en ce cas l'enveloppe (20) comporte au moins deux bords en saillie pour permettre la suture de l'enveloppe (20) elle-même.

15. Sphincter artificiel selon l'une quelconque des revendications précédentes, **caractérisé en ce qu'**il comprend deux ou plusieurs desdits éléments de commutation (13) positionnés et actifs de façon mutuellement parallèle.
